# EUROPEAN PATENT APPLICATION

(11) **EP 3 138 502 A1**
(43) Date of publication of application: **08.03.2017**
(21) Application number: 16184895.7
(22) Date of filing: 19.08.2016
(51) Int. Cl.: A61B 8/08, A61B 8/00, A61B 5/00

(54) **OBJECT INFORMATION ACQUIRING APPARATUS**

(30) Priority: 04.09.2015 US 201562214357 P
(71) Applicant: Canon Kabushiki Kaisha, Tokyo 146-8501 (JP)
(72) Inventor: MASAKI, Fumitaro, Tokyo, Tokyo 146-8501 (JP); SUEHIRA, Nobuhito, Tokyo, Tokyo 146-8501 (JP); FUKUTANI, Kazuhiko, Tokyo, Tokyo 146-8501 (JP); MIYASATO, Takuro, Tokyo, Tokyo 146-8501 (JP); KRUGER, Robert A., Oriental, NC North Carolina 28571 (US)
(74) Representative: TBK

(57) **Abstract**

An object information acquiring apparatus includes a light source, a light absorbing member configured to absorb light radiated from the light source to generate a transmit-ultrasound wave that is a planar wave, a detecting element configured to receive an acoustic wave to output an electric signal, an information processing unit configured to acquire characteristic information on an object using the electric signal. The detecting element receives an ultrasound wave resulting from reflection or scattering of the transmit-ultrasound wave in the object, as an acoustic wave.

## Description

### BACKGROUND OF THE INVENTION

### Field of the Invention

The present invention relates to an object information acquiring apparatus.

### Description of the Related Art

Photoacoustic imaging (PAI) is known as a technique for imaging light absorbers in an object (for example, blood vessels in a living organism). The photoacoustic imaging is a technique for representing the distribution of light absorbers in the form of image data utilizing the fact that, when the object is irradiated with light, photoacoustic waves emanate from the light absorbers based on a photoacoustic effect. For example, utilizing hemoglobin as a light absorber allows blood vessels in the living organism to be imaged.

On the other hand, ultrasound imaging is known as a method for rendering structure information relating to the interior of an object. In the ultrasound imaging, detecting elements (transducers) arranged in a probe transmit ultrasound waves to the object. Then, reflected waves generated at interfaces between acoustic impedances in the object are received and processed into image data.

As ultrasound waves transmitted to the object in the ultrasound imaging, photoacoustic waves generated based on the photoacoustic effect may be used instead of acoustic waves emanating from the detecting elements. In the document Thomas Felix Fehm, Xose Luis Dean-Ben and Daniel Razansky, "Hybrid optoacoustic and ultrasound imaging in three dimensions and real time by optical excitation of a passive element", Proc. of SPIE Vol. 9323, a technique is described in which a handheld probe is used to irradiate point absorbers in the probe with laser light so that the living organism is imaged based on photoacoustic waves resulting from the irradiation.

Non Patent Literature 1: Thomas Felix Fehm, Xose Luis Dean-Ben and Daniel Razansky, "Hybrid optoacoustic and ultrasound imaging in three dimensions and real time by optical excitation of a passive element", Proc. of SPIE Vol. 9323

### SUMMARY OF THE INVENTION

In general, when the light absorber is a point sound source, a photoacoustic wave is isotropically emitted as a spherical wave. Therefore, with increasing distance from the light absorber, the spread of a sound wave increases and the amount of energy per unit area decreases. As a result, upon reaching an examination target, light has reduced energy.

The present invention has been developed to acquire appropriate information on an object using an apparatus that acquires the information using a photoacoustic wave generated based on the photoacoustic effect.

The present invention in its first aspect provides an object information acquiring apparatus as specified in claims 1 to 12.

The present invention allows appropriate information on an object to be acquired using an apparatus that acquires the information using a photoacoustic wave generated based on the photoacoustic effect.

Further features of the present invention will become apparent from the following description of exemplary embodiments with reference to the attached drawings.

### BRIEF DESCRIPTION OF THE DRAWINGS

FIGS. 1A and 1B are diagrams depicting an apparatus configuration in Embodiment 1;
FIG. 2 is a diagram depicting a configuration of a detecting unit and arrangement of detecting elements in Embodiment 1;
FIG. 3 is a diagram illustrating the dependence of the sensitivity of the detecting elements on frequency;
FIG. 4 is a diagram depicting an arrangement configuration for preliminary experiments;
FIGS. 5A and 5B are diagrams illustrating characteristics of a detecting unit determined as a result of the preliminary experiments;
FIG. 6 is a diagram illustrating the dependence, on distance, of outputs obtained as a result of the preliminary experiments;
FIGS. 7A and 7B are diagrams illustrating results of ultrasound imaging of a phantom including a plastic ball;
FIGS. 8A and 8B are diagrams illustrating results of ultrasound imaging of a phantom including wires;
FIGS. 9A and 9B are diagrams depicting a polyethylene sheet evacuated from an optical path;
FIGS. 10A and 10B are diagrams depicting the polyethylene sheets inserted onto the optical path; and
FIGS. 11A to 11C are diagrams depicting an example of insertion and evacuation of a sheet.

### DESCRIPTION OF THE EMBODIMENTS

A preferred embodiment of the present invention will be described below with reference to the drawings. The dimensions, materials, shapes, relative arrangements, and the like of components described below should be changed as needed in accordance with the configuration of an apparatus to which the invention is applied and various conditions for the apparatus. Therefore, the scope of the present invention is not intended to be limited to the following descriptions.

The present invention relates to a technique for detecting an acoustic wave propagating from an object to generate and acquire characteristic information on the interior of the object. Therefore, the present invention is considered to be an object information acquiring apparatus or a control method therefor, or an object information acquiring method or a signal processing method. The present invention is considered to be a program that allows the above-described methods to be executed by an information processing apparatus including hardware resources such as a CPU and memory, or a storage medium that stores the program.

The object information acquiring apparatus includes an apparatus that utilizes the photoacoustic effect to receive a photoacoustic wave generated inside an object irradiated with light (electromagnetic wave) and to acquire characteristic information on the object in the form of image data. The characteristic information in this sense refers to characteristic value information corresponding to a plurality of positions inside the object and generated using reception signals resulting from reception of photoacoustic waves. Such an apparatus may also be referred to as a photoacoustic imaging apparatus.

Characteristics information derived from a photoacoustic wave from the object is values reflecting the absorptivity of light energy. For example, "characteristic information based on light absorption" and "distributions related to an optical characteristic value for the interior of the object" include a source of acoustic waves resulting from light irradiation, the initial sound pressure in the object, or a light energy absorption density or absorption coefficient derived from the initial sound pressure. The characteristic information also includes concentration-related information on a substance forming a tissue.

The concentration-related information includes values that are related to the concentration of a substance present in the object and that are determined using characteristic information based on light absorption for a plurality of wavelengths. Specifically, examples of the concentration-related information include oxygen saturation, a value resulting from weighting of the oxygen saturation with an intensity such as the absorption coefficient, total hemoglobin concentration, oxyhemoglobin concentration, and deoxyhemoglobin concentration. Further examples of the concentration-related information include glucose concentration, collagen concentration, melanin concentration, and the volume factor of fat or water. Based on concentration-related information on different positions in the object, a two- or three-dimensional characteristic information distribution is obtained. Distribution data may be generated in the form of image data. Examples of the characteristic information value distribution include distributions related to the optical characteristic values and the distribution of concentration-related information.

The object acquiring apparatus in the present invention includes an apparatus that utilizes an ultrasound imaging technique to transmit an ultrasound wave to the object and to receive the ultrasound wave reflected or scattered inside the object (reflected scattered wave or echo wave) to acquire object information in the form of image data. Characteristic information derived from the reflected and scattered wave is information reflecting differences in acoustic impedance among tissues inside the object.

The acoustic wave as used herein is an elastic wave that is typically an ultrasound wave and that is also referred to as a sound wave. An electric signal into which an acoustic wave is converted by a transducer or the like is also referred to as an acoustic signal. The term "ultrasound wave" or "acoustic wave" in the following description is not intended to limit the wavelengths of these elastic waves. An acoustic wave resulting from the photoacoustic effect is referred to as a photoacoustic wave.

The object for the object information acquiring apparatus may be a part of a living organism, specifically, a segment of a human being or an animal (a breast, an organ, a circulatory organ, a digestive organ, a bone, muscle, fat, or the like). Examples of a substance to be examined in photoacoustic imaging include hemoglobin, glucose, or water, melanin, collagen, and fat present in the body. Ultrasonic echo examinations allow acquisition of structure information (morphologic information) based on differences in acoustic impedance.

### [Embodiment 1]

Embodiment 1 of the present invention will be described with reference to the drawings. In the following description, a "photoacoustic wave from the object" refers to a photoacoustic wave generated inside the object or by a light absorber on a surface of the object, unless otherwise specified. On the other hand, a photoacoustic wave emanating from a light absorber installed on a laser path outside the object is referred to as a "transmit-ultrasound wave". However, these names are not intended to limit the respective wavelengths. Acquisition of image data on the object using a transmit-ultrasound wave is referred to as "ultrasound wave imaging". Acquisition of image data using a photoacoustic wave from the object is referred to as "photoacoustic imaging".

### (Apparatus Configuration)

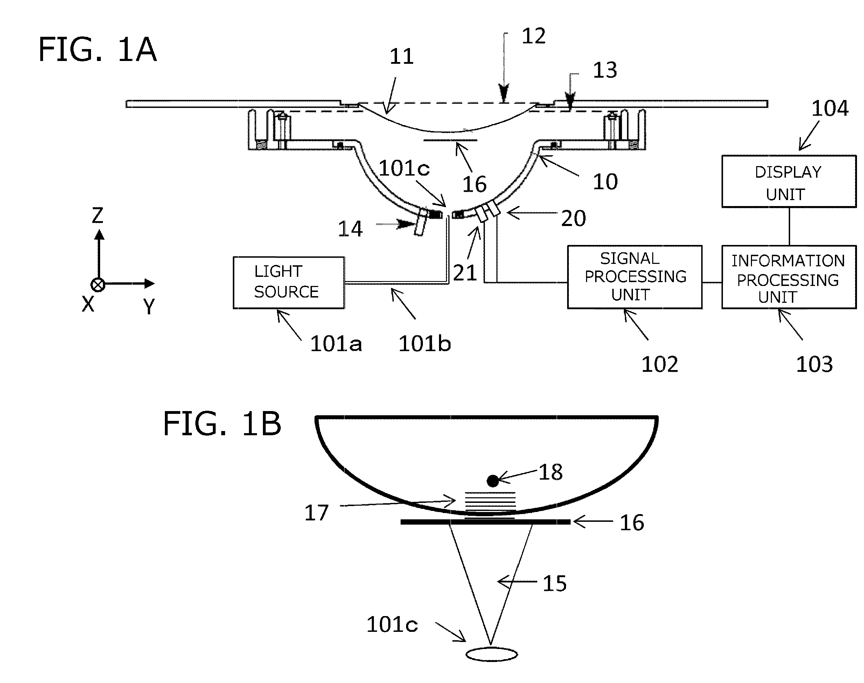

FIG. 1A depicts a sectional view of a main apparatus configuration in the present embodiment. A craniocaudal direction of a subject corresponds to a Y direction. Detection elements are arranged on a curved surface (detection surface) of a bowl-shaped detecting unit 10. For the detecting elements (20 and 21), only one detecting element is illustrated as a representative of each type. The detecting elements (20 and 21) will be described below in detail. An object (for example, a breast of a living organism) is inserted from above and placed in a cup 11 filled with water for acoustic impedance matching. A water surface 12 corresponds to an upper surface of the cup 11. A space between the detection surface and the cup is also filled with water for acoustic impedance matching. The surface of this water is denoted by reference numeral 13. As an acoustic matching material, gel or castor oil may be used instead of water.

The state of the object can be monitored with a camera 14. Optical images taken with the camera can be utilized for various types of control and information processing. A polyethylene sheet 16 is installed below the cup 11 at a predetermined distance therefrom using a sheet support member not depicted in the drawings, such as a frame member or a member based on tension. When the cup is used, the cup is effective for holding the shape of the object to stabilize measurement conditions. When a plurality of polyethylene sheets are prepared and changed according to the object, an attachment unit may be provided which allows the sheet as a whole including the support member to be changed. Alternatively, as the support member, a clip or a protruding portion may be used which allows the sheet to be fixed.

Light from a light source 101a is radiated through an emission end 101c via an optical system 101b. A suitable light source is a pulse laser apparatus (for example, a Ti:S laser with a wavelength of approximately 800 nm). However, a flash lamp or a light emitting diode may also be utilized. For an apparatus using both ultrasound imaging and photoacoustic imaging as described below, the wavelength of radiated light is determined according to the absorption characteristic or absorption spectrum of the light absorber. The oxygen saturation or the like can be measured by utilizing, as a light source, a wavelength varying laser that allow light with a plurality of wavelengths to be radiated.

The optical system 101b is provided to radiate light with a desired shape and a desired intensity. As the optical system, for example, optical fibers, lenses, mirrors, prisms, diffusers, or the like can be utilized. In FIG. 1A, the emission end 101c is provided at the center of a bottom portion of the bowl. However, the emission end 101c is provided at any position so long as the polyethylene sheet 16 can be irradiated with light through the emission end 101c.

Upon receiving an ultrasound wave (acoustic wave), each of the detecting elements (20 and 21) outputs an analog electric signal. A signal processing unit 102 executes amplification, digital conversion, or a correction process, on the analog electric signal as needed, and outputs the resultant signal. The output digital electric signal is input to the information processing unit 103 directly or via a memory (not depicted in the drawings). The information processing unit is an information processing apparatus including computational resources such as a CPU and a storage apparatus, and may be, for example, a processing circuit, a workstation, or a PC. The information processing unit reconstructs the digital electric signal into image data, and outputs the image data to a display unit 104. For the image reconstruction, known techniques such as phasing addition, Fourier transform, or back projection may be utilized. The display unit may be provided separately from the object information acquiring apparatus. As the display unit, a display apparatus such as a liquid crystal display or a plasma display may be utilized.

### (Generation of a Transmit-Ultrasonic Wave)

FIG. 1B is a schematic view illustrating generation of a transform-ultrasound wave. The polyethylene sheet 16 having absorbed laser light 15 radiated through the emission end 101c generates a planar transmit-ultrasound wave 17 based on the photoacoustic effect. As a material for the polyethylene sheet 16, a high-density polyethylene sheet is suitable which contains a black paint. When the sheet is formed to have a thickness of approximately 0.1 mm and is installed on the laser optical path, nearly 100% of the laser light is absorbed.

Any member for generating a transmit-ultrasound wave (light absorbing member or a planar-wave generating member) may be used so long as the member has a function to absorb light radiated from the light source to generate a planar sound wave. That is, besides the polyethylene sheet, various materials such as acrylic, polyester, and polyvinyl chloride are available. In a positional relation in which the light absorbing member (planar-wave generating member) is located between the detecting unit and the object as illustrated in FIGS. 1A and 1B, the light absorbing member (planar-wave generating member) is preferably formed of a material that allows an ultrasound wave reflected and scattered inside the object to pass through.

A suitable material for the light absorber contained in the sheet is carbon. However, an alternative pigment may be used. Furthermore, an absorber in a color other than black may be used. The thickness of the sheet is determined according to a desired frequency of the transmit-ultrasound wave. In general, the frequency of the transmit-ultrasound wave decreases with increasing thickness of the sheet and increases with decreasing thickness of the sheet. The sound pressure of the transmit-ultrasound wave is increased by increasing the concentration of the pigment or the thickness of the sheet. Thus, the sheet may be changed according to the object. The frequency of the transmit-ultrasound wave is varied by varying the pulse width of the laser. The frequency of the transmit-ultrasound wave decreases with increasing pulse width of the laser and increases with decreasing pulse width of the laser.

The transmit-ultrasound wave propagates inside the living organism and is reflected and scattered at an interface inside the living organism which involves a difference in acoustic impedance. A reflector/scatterer may be, for example, a fine calcareous corpuscle 18 in the breast. In the present embodiment, the bowl-shaped detecting unit receives a reflected and scattered ultrasound wave derived from a planar transmit-ultrasound wave emanating from the polyethylene sheet 16 as described above. Thus, an area image with a certain size can be collectively acquired (during a single transmission or reception of a planar wave).

### (Detecting Unit and Detection Elements)

Now, a configuration of the detecting unit 10 will be described. In an apparatus that reconstructs a reflected and scattered ultrasound wave from the interior of the object to generate a three-dimensional image of the object, the detecting elements are desirably arranged on a closed curved surface that surrounds the object in order to accurately determine a sound pressure distribution. In particular, if a plurality of detecting elements can be arranged on a spherical surface (or a semi-spherical surface), acoustic waves can be evenly detected in all directions, enhancing homogeneity of reconstructed image contrast. However, the detecting unit may be a primary probe or a secondary planar probe.

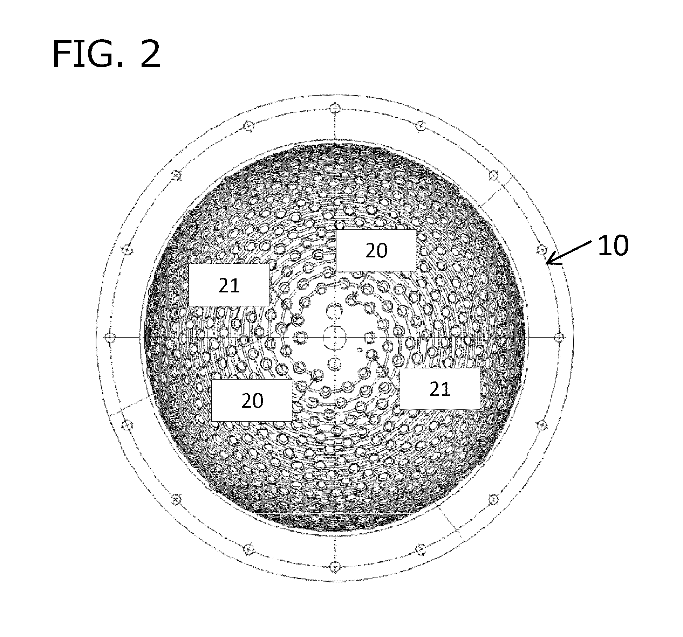

FIG. 2 is a diagram of the detection surface as viewed from above (from a cup side). The plurality of detecting elements (transducers) 20 and 21 are provided on the detection surface. The plurality of detecting elements 20 are spirally arranged and have a central frequency of 2 MHz. The plurality of detecting elements 21 are spirally arranged and have a central frequency of 5 MHz. The detecting elements may be piezoelectric elements, capacitive micromachined ultrasonic transducers (cMUTs), polyvinylidene difluoride (PVDF), or Fabry-Perot sensors.

In general, detecting elements for ultrasound waves are capable of receiving only ultrasound waves in a narrow frequency band of approximately ±50% from the central frequency. Thus, in the present embodiment, two types of detecting elements (20 and 21) having different central frequencies are rotation-symmetrically arranged on the detection surface. This enables ultrasound waves to be received over a wide frequency band and facilitates processing for three-dimensional reconstruction.

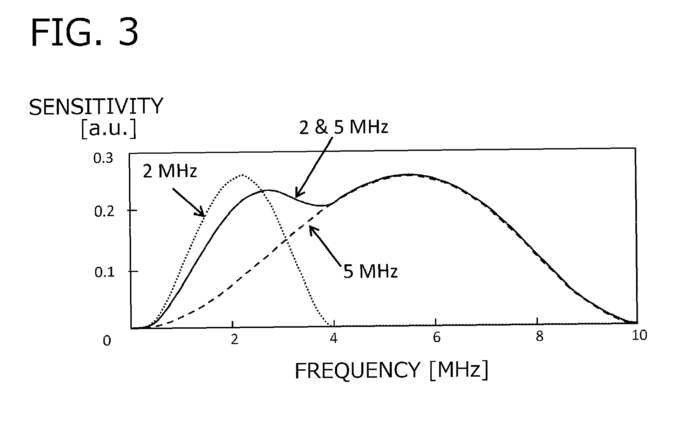

FIG. 3 illustrates a frequency band that can be covered by a combination of the two types of detecting elements having different central frequencies. The sensitivity of the detecting elements 20 is depicted by a dotted line, and the sensitivity of the detecting elements 21 is depicted by a dashed line. A solid line corresponds to a band that can be covered by the combination of the detecting elements 20 and 21. Even if the sensitivity of the detecting elements varies according to frequency region, a relatively even sensitivity can be obtained over a wide frequency band by adjusting the gain of output obtained during electric-signal conversion. For example, in FIG. 3, the high-frequency detecting elements have a low frequency, and thus, the output gain of the high-frequency detecting elements during electric-signal conversion may be increased (for example, doubled) during the electric-signal conversion. A gain controlling unit not depicted in the drawings performs such adjustment of the output gain to enable the sensitivities of the detecting elements to be made equal. An existing gain controlling circuit can be utilized as the gain controlling unit. Three or more types of detecting elements may be used.

In an ultrasound apparatus including a linear probe, a plurality of (for example, 128) detecting elements transmit an ultrasound wave to an examination target and receive the ultrasound wave reflected inside the examination target. Such an ultrasound apparatus can capture a large structure such as a tumor interface but has difficulty capturing a small structure such as a calcareous corpuscle. This is because the large interface regularly reflects and returns the ultrasonic wave to an ultrasound transducer, whereas the small structure scatters the ultrasound wave at various angles and returns a reduced amount of ultrasound wave to the ultrasound transducer. However, arrangement of the detecting elements in the bowl-shaped (semi-spherical) container enables even an ultrasound wave scattered by the calcareous corpuscle in the breast to be received.

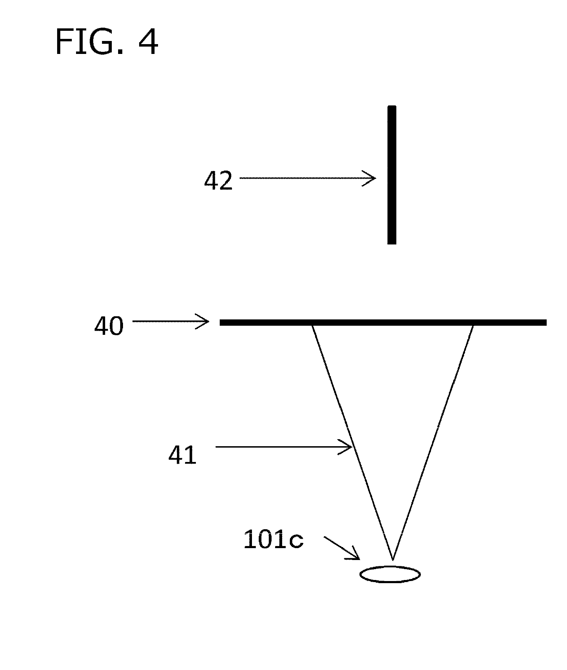

FIG. 4 depicts a configuration of an arrangement for preliminary experiments for measurement of characteristics of a generated transmit-ultrasound wave. A polyethylene sheet 40 placed under water was irradiated with laser light 41, and a resultant ultrasound wave was detected with a hydrophone 42.

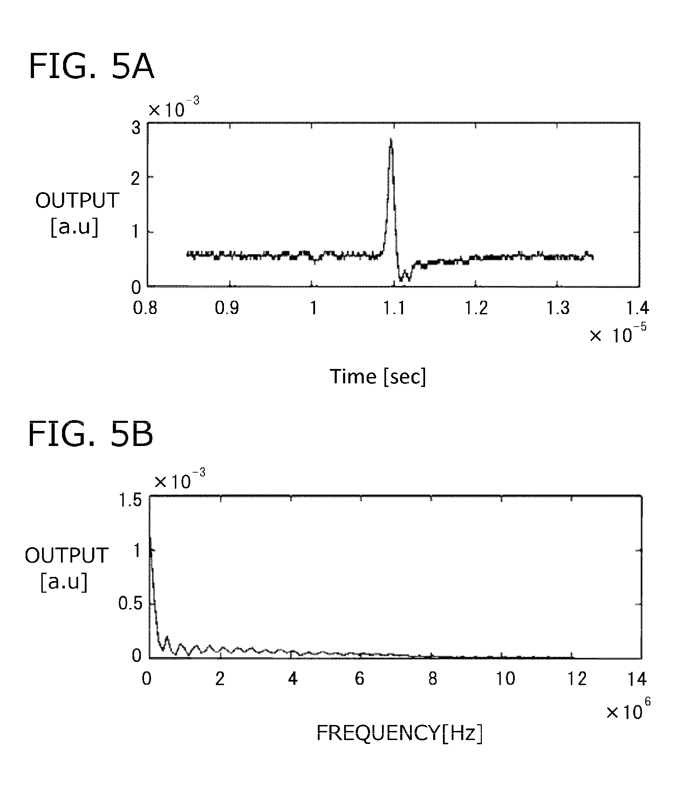

FIG. 5A illustrates the dependence, on time, of the signal intensity of an actually measured transmit-ultrasound wave. The transmit-ultrasound wave has a sharp peak. FIG. 5B illustrates frequency components of the transmit-ultrasound wave. As illustrated in FIG. 5B, the transmit-ultrasound wave has a wide frequency band from low frequency to high frequency (approximately 0.1 MHz to approximately 8 MHz). For example, if a piezoelectric element is used as a transmission source of sound waves, the piezoelectric element can transmit only sound waves in a narrow frequency band of approximately ±50% from the central frequency. On the other hand, when the transmit-ultrasound wave is generated in accordance with the method in the present embodiment and the above-described two type of detecting elements are used, ultrasound waves in a wide frequency band can be transmitted and received. As a result, it can be expected that image quality is improved and that more targets in the living organism can be covered.

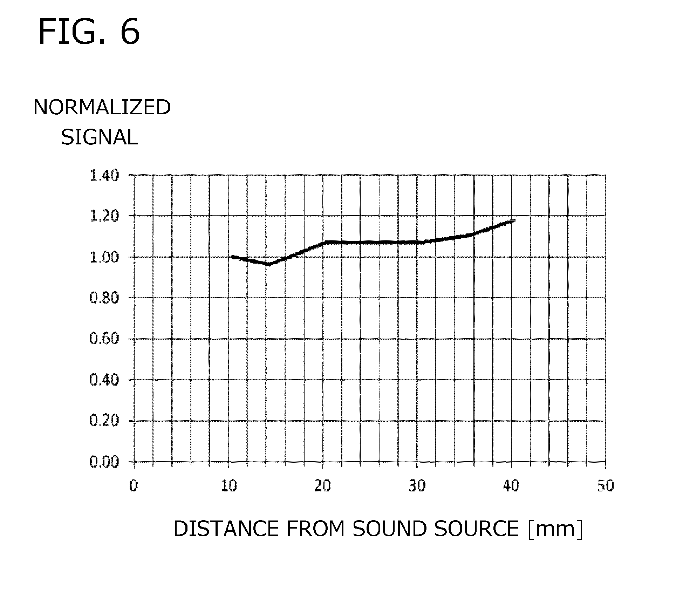

The resultant transmit-ultrasound wave is a planar wave, and thus, energy density (sound pressure intensity) is prevented from decreasing even at a position away from the sheet. The dependence of sound pressure intensity on distance will be described using FIG. 6. FIG. 6 illustrates a relation between the sound pressure of a planar wave emanating from the polyethylene sheet and the distance between the hydrophone and the polyethylene sheet, in which the pressure and the distance were measured using the above-described preliminary experiment apparatus. The axis of ordinate represents the sound pressure of a planar wave emanating from the polyethylene sheet, and indicates the intensity of a normalized signal. The axis of abscissas represents the distance between the sound source and the hydrophone (mm).

FIG. 6 indicates that the signal intensity does not decrease even at a position approximately 40 mm away from the sheet. In general, when the object is the breast, signals with the signal intensity thereof prevented from decreasing at a distance of approximately 40 mm are sufficient for measurement. When an area of interest is a deep portion of the object, a semi-spherical polyethylene sheet may be used. In this case, the transmit-ultrasound wave converges toward the center of curvature of the polyethylene sheet, and is thus suitable for imaging of reflectors/scatterers located in the vicinity of the center of the curvature.

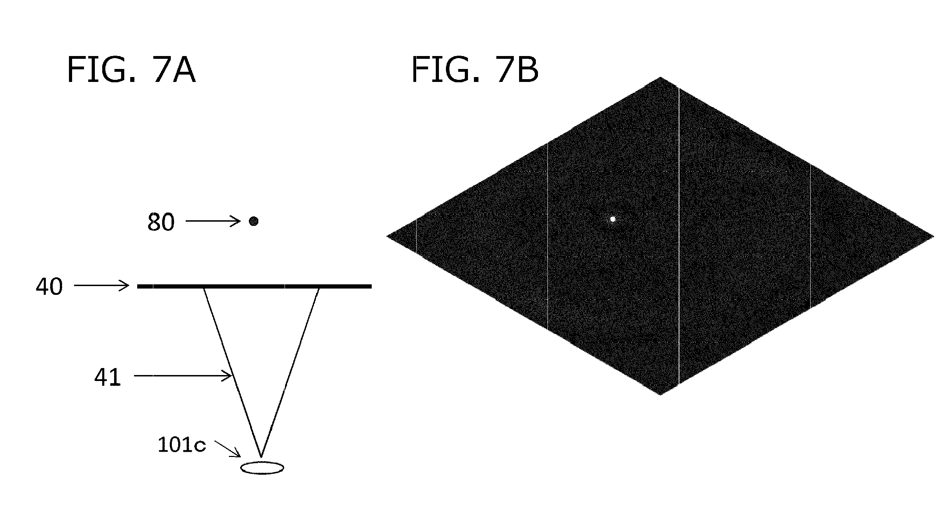

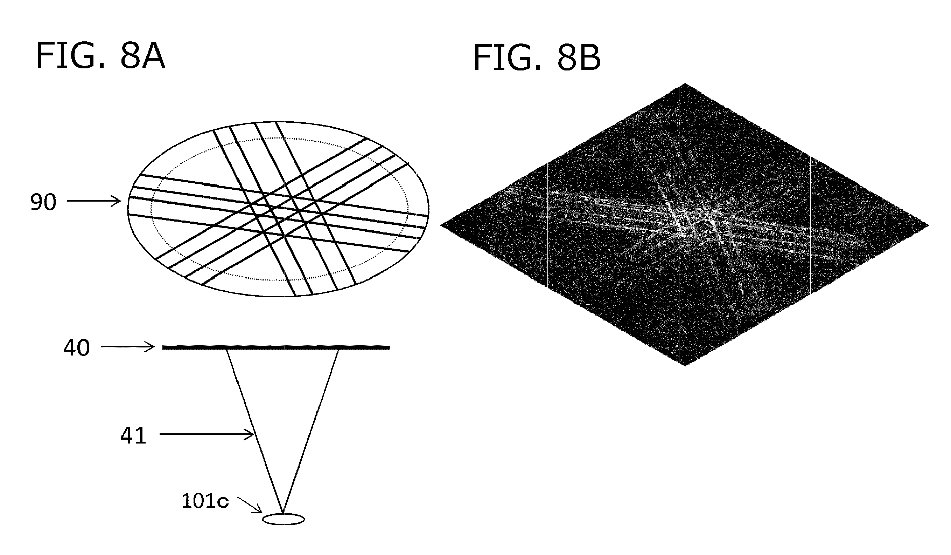

FIG. 7B depicts the results of imaging of a point scatterer (a plastic sphere 80 depicted in FIG. 7A) installed in a phantom simulating the living organism. FIG. 8B depicts the results of imaging of a wire scatterer (12 wires 90 depicted in FIG. 8A) instead of the point scatter. Each of the scatterers can be fixed in position using any support mechanism such as a frame member, wires, or a support film. As depicted in FIGS. 7B and 8B, a planar transmit-ultrasound wave generated based on the photoacoustic effect as in the present embodiment can be used to generate image data indicative of characteristic information on the object.

To enlarge an imaging area, a scanning mechanism (scanning unit) is preferably provided. Since the transmit-ultrasound wave is a planar wave, an area outside the area irradiated with the transmit-ultrasound wave is precluded from being imaged. The size of the area is determined in association with the size of the polyethylene sheet. However, the imaging area can be enlarged by scanning the detection surface, the emission end, and the polyethylene sheet with respect to the object. As the scanning mechanism, any of various known mechanisms can be utilized, and for example, an XY stage is preferable. A scanning trajectory is suitably spiral, but a raster scan scheme with a main scanning direction and a sub-scanning direction may be used.

### [Embodiment 2]

In Embodiment 1 described above, the photoacoustic wave is generated so as to be utilized as the transmit-ultrasound wave. That is, image data on the object is obtained by ultrasound imaging. The apparatus in the present embodiment acquires image data based on photoacoustic imaging in addition to image data based on ultrasound imaging.

The photoacoustic imaging needs a mechanism that irradiates the object with light from the light source. As such a mechanism, a light source that allows transmit-ultrasound waves to be generated may be used or a light source different from the light source for transmit-ultrasound waves may be installed. For the mechanism for transmit-ultrasound waves and the mechanism for photoacoustic imaging, the same light source may be used with different optical systems and emission ends provided. If the light source for generation of transmit-ultrasound waves is used as the above-described mechanism, when the light absorbing member (planar-wave generating member) such as the polyethylene sheet is located on the optical path from the emission end to the object, arrival of a sufficient amount at the object is prevented. Thus, in the present embodiment, the polyethylene sheet is evacuated from the laser optical path during the photoacoustic imaging.

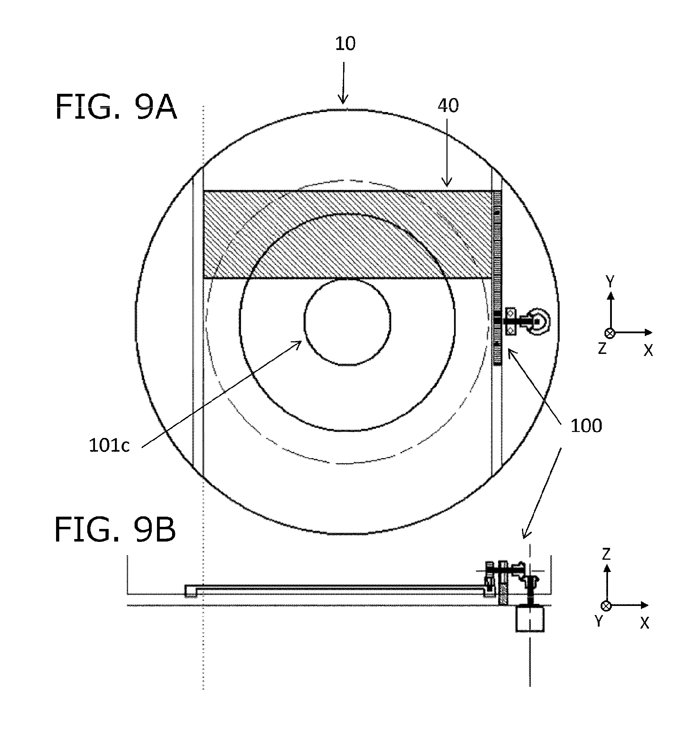

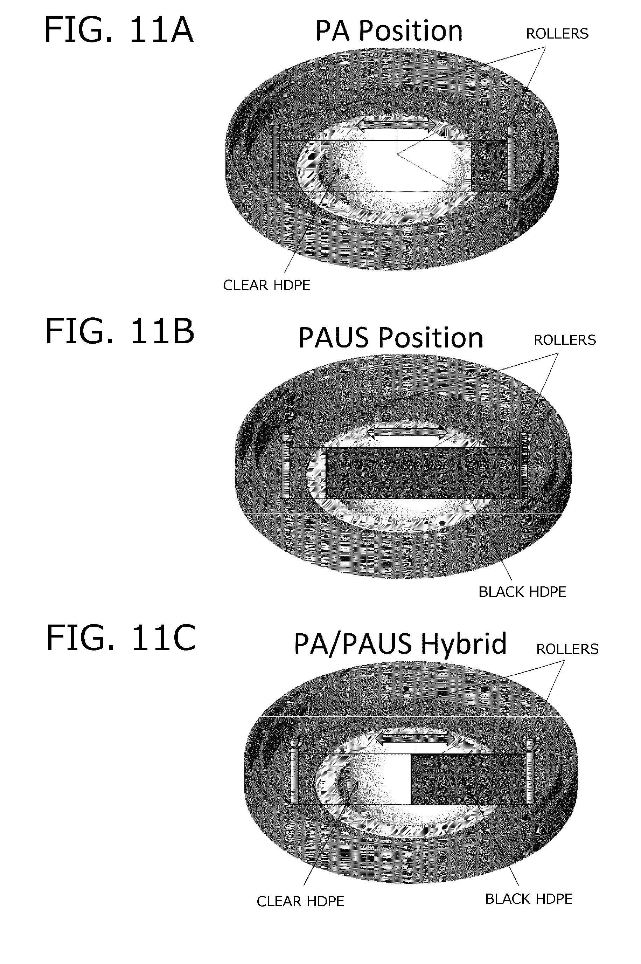

FIGS. 9A and 9B depict an evacuating mechanism 100. FIG. 9A illustrates that the polyethylene sheet 40 has been evacuated from the laser optical path (a state where photoacoustic waves are generated). As depicted in FIGS. 9A and 9B, in the present embodiment, the polyethylene sheet 40 moves away from the optical path in the XY plane. However, a method for evacuating the sheet away from the optical path is not limited to this. For example, the method may involve rolling the sheet (FIGS. 11A to 11C), laying and raising a sheet with a certain level of strength, or using a rotating table. Any of various evacuating mechanisms may be used including a mechanism that includes gears and screws, a mechanism manually operated by a user, and a mechanism that includes a motor.

In such an evacuating state, when laser light is radiated through the emission end 101c, a sufficient amount of light reaches the object. As a result, a photoacoustic wave emanates from a light absorber in the object. For example, when a laser with a wavelength of approximately 800 nm is used, hemoglobin, which exhibits a high light absorption coefficient in this wavelength region, is a dominant light absorber. Thus, a blood vessel structure can be imaged. Each of the detecting elements on the detection surface converts the photoacoustic wave from the object into an electric signal. The information processing unit performs image reconstruction based on the electric signal to acquire photoacoustic image data.

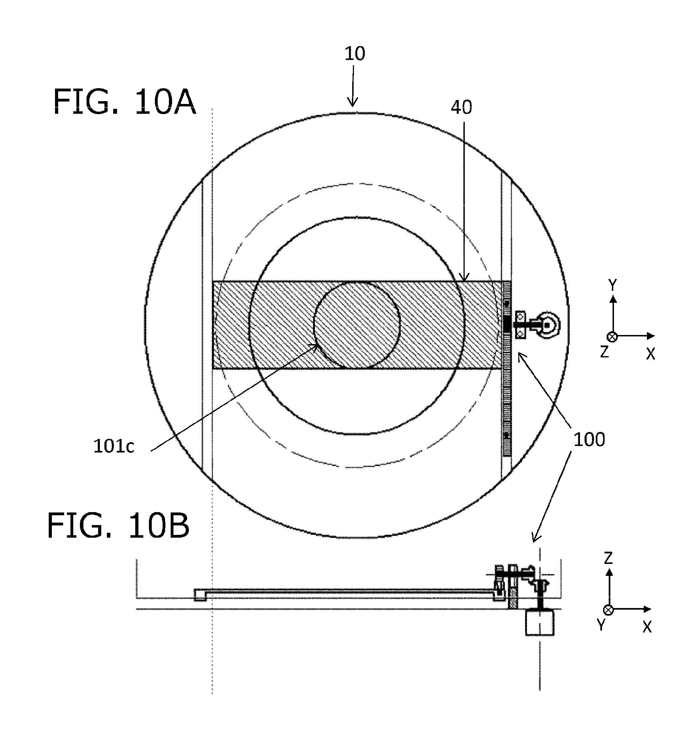

FIGS. 10A and 10B illustrate that the polyethylene sheet 40 has been inserted onto the optical path (a state where transmit-ultrasound waves are generated). In this state, when laser light emitted through the emission end 101c is absorbed by the polyethylene sheet, a planar transmit-ultrasound wave is generated. A portion of the transmit-ultrasound wave reaches the object and is reflected and scattered at an interface between acoustic impedances. As a result, a scatterer/reflector inside the object (for example, a fine calcareous corpuscle in the breast) can be imaged.

A technique similar to ultrasound imaging based on transmit-ultrasound waves is applicable to reconstruction of photoacoustic waves from the object. In this case, time is taken into account which includes a time needed for a transmit-ultrasound wave emanating from the polyethylene sheet 40 to propagate to the reflector/scatterer and a time needed for the transmit-ultrasound wave to propagate from the reflector/scatterer to the detecting element. Using the same reconstruction algorithm for the photoacoustic imaging and for the ultrasound imaging is effective for reducing development loads and simplifying the apparatus.

As described above, simple addition of a simple mechanism for inserting and evacuating the polyethylene sheet allows a single apparatus to achieve both photoacoustic imaging and ultrasound imaging. As a result, both a fine calcareous corpuscle and the blood vessel structure can be imaged.

The area reconstructed by photoacoustic imaging is limited to the area reached by laser light. Thus, to widen the imaging area, the area on the object irradiated with light may be moved. For example, when the emission end is provided at the center of the bowl-shaped (semi-spherical) detecting unit, scanning the detecting unit allows the light irradiation area to be simultaneously moved. This widens the imaging area. A noise reduction effect is produced for areas subjected to duplicate measurement. A scanning mechanism for photoacoustic imaging can also be used for ultrasound imaging.

Image data resulting from photoacoustic imaging may be stored in a storage apparatus not depicted in the drawings or displayed on the display unit 104. When both an image resulting from ultrasound imaging and an image resulting from photoacoustic imaging are displayed, any technique may be utilized such as superimposed display, parallel display, or time difference display. These images may be superimposed on an optical image taken with the camera 14.

### Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.
An object information acquiring apparatus includes a light source, a light absorbing member configured to absorb light radiated from the light source to generate a transmit-ultrasound wave that is a planar wave, a detecting element configured to receive an acoustic wave to output an electric signal, an information processing unit configured to acquire characteristic information on an object using the electric signal. The detecting element receives an ultrasound wave resulting from reflection or scattering of the transmit-ultrasound wave in the object, as an acoustic wave.

## Claims

1. An object information acquiring apparatus comprising:
a light source;
a light absorbing member configured to absorb light radiated from the light source to generate a transmit-ultrasound wave that is a planar wave;
a detecting element configured to receive an acoustic wave and output an electric signal; and
an information processing unit configured to acquire characteristic information on an object using the electric signal,
wherein the detecting element receives an ultrasound wave resulting from reflection or scattering of the transmit-ultrasound wave in the object, as the acoustic wave.

2. The object information acquiring apparatus according to claim 1, further comprising a detecting unit that supports a plurality of the detecting elements,
wherein the light absorbing member is arranged between the object and the detecting elements.

3. The object information acquiring apparatus according to claim 2, wherein the plurality of detecting elements include a plurality of types of detecting elements having different central frequencies.

4. The object information acquiring apparatus according to claim 3, further comprising a gain controlling unit configured to adjust output gains of the plurality of types of detecting elements.

5. The object information acquiring apparatus according to any one of claims 1 to 4, wherein the light absorbing member is a sheet containing a pigment.

6. The object information acquiring apparatus according to claim 5, wherein the sheet is a polyethylene sheet containing carbon as the pigment.

7. The object information acquiring apparatus according to any one of claims 1 to 6, further comprising an evacuating mechanism configured to move the light absorbing member between a position on an optical path from the light source to the object and a position located away from the optical path.

8. The object information acquiring apparatus according to claim 7, wherein the detecting element receives, as the acoustic wave, a photoacoustic wave emanating from the object having absorbed light radiated from the light source when the light absorbing member is positioned away from the optical path.

9. The object information acquiring apparatus according to claim 8, wherein the information processing unit performs ultrasound imaging in which image data is generated using an electric signal based on the transmit-ultrasound wave and photoacoustic imaging in which image data is generated using an electric signal based on the photoacoustic wave from the object.

10. The object information acquiring apparatus according to claim 9, further comprising a display unit configured to display the image data obtained by the ultrasound imaging and the image data obtained by the photoacoustic imaging.

11. The object information acquiring apparatus according to claim 10, further comprising a camera configured to acquire an optical image of the object,
wherein the display unit superimposes the image data on the optical image for display.

12. The object information acquiring apparatus according to any one of claims 1 to 11, further comprising a scanning unit configured to move the detecting element, the light absorbing member, and an emission end through which light from the light source is emitted, with respect to the object.
